# EUROPÄISCHE PATENTSCHRIFT

(11) **EP 2 321 400 B1**
(45) Veröffentlichungstag und Bekanntmachung des Hinweises auf die Patenterteilung: **14.11.2012**
(21) Anmeldenummer: 09782707.5
(22) Anmeldetag: 07.09.2009
(51) Int. Cl.: C12M 1/14

(54) **VORRICHTUNG ZUR HERSTELLUNG BAKTERIELL SYNTHETISIERTER CELLULOSE ODER CELLULOSEHALTIGEM FLÄCHENMATERIAL**
APPARATUS FOR THE PRODUCTION OF BACTERIALLY SYNTHESIZED CELLULOSE OR CELLULOSE-CONTAINING PLANAR MATERIAL
DISPOSITIF POUR LA PRODUCTION PAR SYNTHÈSE BACTÉRIENNE DE CELLULOSE OU DE MATÉRIAU EN NAPPE CONTENANT DE LA CELLULOSE

(30) Priorität: 09.09.2008 DE 102008046298
(43) Veröffentlichungstag der Anmeldung: 18.05.2011
(73) Patentinhaber: EPC Engineering Consulting GmbH, 07407 Rudolstadt (DE)
(72) Erfinder: SCHMIDT, Wolfgang, 07318 Saalfeld (DE); HENKEL, Nadine, 98746 Schwarzmühle (DE); ERDMANN, Rainer, 07407 Rudolstadt (DE); KRALISCH, Dana, 07749 Jena (DE); HESSLER, Nadine, 96529 Mengersgereuth-Hämmern (DE)
(74) Vertreter: Kruspig, Volkmar
(86) Internationale Anmeldenummer: PCT/EP2009/061568
(87) Internationale Veröffentlichungsnummer: WO 2010/029044

(56) Entgegenhaltungen:
- WO-A1-2004/050986
- WO-A1-2006/066377
- DE-A1- 4 310 100
- DE-U1- 20 309 734
- GB-A- 194 623
- JP-A- 6 253 877
- JP-A- 8 033 495
- JP-A- 10 195 713
- JP-A- 2005 082 704
- US-A- 6 071 727
- DATABASE WPI Week 199937 Thomson Scientific, London, GB; AN 1999-440781 XP002611147, & JP 11 182435 A (ORION KIKAI KK) 6. Juli 1999 (1999-07-06)
- DATABASE WPI Week 199538 Thomson Scientific, London, GB; AN 1995-288157 XP002611148, & JP 7 185245 A (FUKUHARA KK) 25. Juli 1995 (1995-07-25)

## Beschreibung

Die Erfindung betrifft eine Vorrichtung zur Herstellung bakteriell synthetisierter Cellulose oder cellulosehaltigem Flächenmaterial, umfassend ein wannenartiges Reaktionsgefäß zur Aufnahme einer Nährlösung, Mittel zur Nährlösungszuführung und Temperierung des Reaktionsgefäßes sowie zur Sauerstoffversorgung gemäß Patentanspruch 1.

Bakteriell synthetisierte Cellulose, auch Bakteriencellulose (BC) genannt, wird üblicherweise in Standkultivierung mit einem Nährmedium gewonnen. Das gebildete Material wird an der Grenzfläche zwischen Luft und Nährmedium abgeschöpft. Hierbei ist jedoch die Größe des gewonnenen Vlieses von den Abmessungen des Kultivierungsgefäßes abhängig und somit begrenzt.

Weiterhin sind Lösungen bekannt, welche diese Abhängigkeit der Form des Bakteriencellulose-Materials vom Kultivierungsgefäß vermeiden sollen. Gemäß S. Tokura, H. Tamura et al: Continuous Harvest of Cellulosic Filament during Cultivation of Acetobacter xylinum, Cellulosic Pulps, Fibres and Materials; International Cellucon Conference (on) Pulp for Papermaking; Fibre and Surface Properties and Other Aspects of Celluluse Technology, 10t h, Turku/Abo, Finland 2000, Meeting Date 1998, besteht die Möglichkeit der Herstellung von BC-Fasern in einem flachen Becken unter Standkultivierung bei kontinuierlicher Aufwicklung der an der Oberfläche des Kulturmediums synthetisierten Filamente.

Eine ähnliche Lösung ist auch in der JP 10195713 offenbart. Diese Druckschrift zeigt eine Vorrichtung zur Herstellung bakteriell synthetisierter Cellulose, welche ein wannenartiges langgestrecktes Reaktionsgefäß zur Aufnahme einer Nährlösung umfasst. Zudem verfügt die aus der Druckschrift JP 10195713 bekannte Vorrichtung über Mittel zur Nährlösungszuführung und Temperierung des Reaktionsgefäßes sowie zur Sauerstoffversorgung.

Es ist weiterhin bekannt, eine Steigerung der Biosynthese durch eine gleichzeitige Sauerstoffzufuhr zu erreichen. Hierbei wird jedoch keine Bakteriencellulose in flächiger Form gebildet, sondern es entstehen lediglich zusammengelagerte Filamente mit uneinheitlicher, inhomogener innerer Struktur.

Gemäß US 6,071,727 ist ein Bioreaktor vorbekannt, der mit einem Förderband versehen ist, das kontinuierlich durch das Nährmedium geleitet wird.

Hier werden partiell getrocknete BC-Folien aus einem BC-Gelfilm erhalten. Auch bei dieser Lösung entsteht nur ein sehr inhomogenes Material ohne definierte Abmessungen.

Die JP 2005082704 zeigt die Aufwicklung von an der Grenzfläche zwischen Luft und Nährmedium kultivierten BC-Biofilmen unter Bildung flächiger Vliese. Hierbei wird jedoch die Bakteriencellulose einer mechanischen Kraft ausgesetzt und dadurch die Struktur verändert. Zudem erfolgt die Aggregation des Biofilms zur dünnen Folie nicht während der Biosynthese im Reaktor, sondern durch eine vorgegebene Anordnung der synthetisierten Schichten während des Aufwicklungsprozesses. Es lassen sich auf diese Weise keine Vliese mit homogener, dreidimensionaler Netzwerkstruktur erzielen.

Eine Standkultivierung hat darüber hinaus den Nachteil, dass sie sehr flächenintensiv ist und sich nicht für eine großtechnische Produktion eignet. Auch kann keine gleichbleibende, optimale Versorgung an Nährmedien gewährleistet werden. Damit ist die Effizienz derartiger Verfahren geringer als bei einer kontinuierlichen Versorgung mit Nährmedium bei optimaler Verfügbarkeit der Nährmediumsinhaltsstoffe.

Eine Methode zur semi-kontinuierlichen Herstellung von Bakteriencellulose ist in der JP 06253877 erläutert. Das dort gewonnene Vlies entspricht jedoch nur der Reaktorgeometrie. Variable Vliesabmessungen können nicht realisiert werden.

Bekannt ist ebenfalls eine kontinuierliche Abtrennung der synthetisierten Bakteriencellulose im Rahmen eines Fermentationsprozesses, wodurch die Konzentration im Reaktor gering gehalten wird. Diesbezüglich sei auf die JP 08033495 verwiesen.

Die WO 2004/050986 betrifft eine Methode, bei welcher das Nährmedium mittels eines mechanischen Mixers hergestellt, in Kästen gegeben und mit recycelter oder nichtrecycelter Vorkultur beimpft wird. Die Temperatur der Anlage wird durch eine Ummantelung konstant gehalten, so dass bei Bakteriencellulose-Folien hergestellt werden können.

Gemäß der WO 2006/066377 wird ein erhaltenes Bakteriencellulose-Vlies entwässert und dann die Kultivierung fortgesetzt. Dies kann so lange durchgeführt werden, bis das Volumen nur noch 15% bis 25% des Ausgangsvolumens aufweist. Danach ist es notwendig, das Nährmedium zu erneuern. Es bleibt jedoch bei dieser Lösung der Nachteil bestehen, dass die gewonnene Fläche an bakteriell synthetisierter Cellulose von der Form der verwendeten Reaktionsgefäße abhängt und somit einerseits in der Größe limitiert und andererseits nicht variabel ist. Ebenfalls schwankt die Nährmediumskonzentration über die Zeitdauer des Verfahrens. Hierdurch ist keine gleichbleibende, optimale Nährmittelverfügbarkeit gegeben.

Aus dem Vorgenannten ist es daher Aufgabe, eine weiterentwickelte Vorrichtung zur Herstellung bakteriell synthetisierter Cellulose oder cellulosehaltigem Flächenmaterial anzugehen, mit deren Hilfe, das Herausheben und Transportieren des Flächenkörpers aus dem Reaktionsgefäß kräftefrei möglich ist und zwar derart, dass die gewonnene synthetische Zellulose nicht beschädigt wird.

Die Lösung der Aufgabe der Erfindung erfolgt durch eine Vorrichtung gemäß der Merkmalskombination nach Patentanspruch 1, wobei die Unteransprüche zweckmäßige Ausgestaltungen und Weiterbildungen beinhalten.

Mit Hilfe der erfindungsgemäßen Vorrichtung gelingt es, die Voraussetzung für ein universell anwendbares und für eine großtechnische Produktion geeignetes Herstellungsverfahren zu schaffen, welches eine effiziente Produktion homogener, flächiger, bakteriell synthetisierter Cellulose sowie cellulosehaltiger Materialien in einer von der Reaktorgeometrie unabhängigen sowie beliebig wählbaren definierten Länge und Dicke ermöglicht.

Es wird also von einer Vorrichtung zur Herstellung bakteriell synthetisierter Cellulose oder cellulosehaltigem Flächenmaterial ausgegangen, welche ein wannenartiges Reaktionsgefäß zur Aufnahme einer Nährlösung, Mittel zur Nährlösungszuführung und Temperierung des Reaktionsgefäßes sowie zur Sauerstoffversorgung umfasst und das Reaktionsgefäß einen langgestreckten Wannenkörper mit einem ersten und einem zweiten Ende aufweist.

Am ersten Ende ist ein Zulauf für eine inhaltsreiche Nährstofflösung und am zweiten, gegenüberliegenden Ende ein Ablauf für inhaltsarme Nährstofflösung vorgesehen.

Am zweiten Ende des Wannenkörpers ist eine Gleitschräge oder ein Gleitschrägkörper für das abzuziehende Flächenmaterial ausgebildet.

Außerhalb des zweiten Endes des Wannenkörpers befindet sich ein Abzugsund Fördermodul, welcher mindestens ein motorisch angetriebenes Band sowie eine Spül- und Reinigungswanne für das abgezogene Flächenmaterial aufweist.

Erfindungsgemäß ist mindestens der Zulauf für die inhaltsreiche Nährstofflösung unterhalb des üblichen Nährstofffüllspielgels im Wannenkörper befindlich.

Zudem weist erfindungsgemäß der Abzugs- und Fördermodul einen verstellbaren Abzugsarm auf, welcher an seinem zur Wanne reichenden Ende eine Abzugswalze oder mindestens ein Abzugsritzel besitzt.

Dabei steht die Walze oder das Ritzel mit einer Antriebseinrichtung in Verbindung. Des Weiteren nimmt die Walze oder das Ritzel einen unterhalb des Flächenmaterials befindlichen, flexiblen, flächigen Produktstützkörper auf und/oder kontaktiert diesen zum Zweck des Transports. Hierdurch ist die gewünschte kräftefreie Möglichkeit des Heraushebens und Transportierens des Flächenkörpers aus dem Reaktionsgefäß möglich.

Ausgestaltend umfasst der Zulauf einen Verteilkörper zum Erzeugen eines definierten, möglichst gleichmäßigen Nährstofflösungs-Volumenstroms.

Dieser Verteilkörper ist bevorzugt am Wannenboden befindlich und weist einen in Wannenquerrichtung verlaufenden Steg mit Bohrungen und Schlauchanschlussstutzen auf.

Die Gleitschräge oder der eine Gleitschräge umfassende Körper besitzt eine Schrägfläche, deren Verlauf sich von unterhalb des Nährstofffüllspiegels bis zu einer schlitzartigen Öffnung in der Wanne oberhalb des Nährstofffüllspiegels erstreckt.

Diese Gleitschräge ermöglicht ein sicheres, von mechanischen Kräften nahezu freies Abheben und Transportieren des Vlies-Flächenmaterials.

Der Stützkörper ist beispielsweise als ein eigensteifes Netz ausgebildet.

Mindestens der Wannenkörper ist von einer Umhausung umgeben, welche mindestens in ihren Breitenabmessungen größer als der Wannenkörper ist, wobei im jeweiligen seitlichen Zwischenraum Lochrohre oder Lochschläuche zur turbulenzarmen Zuführung von Reaktionsluft vorhanden sind.

Im, am und/oder unterhalb des Wannenkörpers sind Mittel zum Beheizen desselben vorgesehen.

Diese Mittel zum Beheizen können eine Heizmatte, insbesondere mit elektrischen Widerstandsheizleitern umfassen.

Das motorisch angetriebene Band des Abzugs- und Fördermoduls kann als Endlos-Noppenband ausgeführt werden.

Weiterhin sind erfindungsgemäß Mittel zum Messen der Sauerstoffkonzentration sowohl in der Reaktionsluft als auch in der Reaktionslösung vorgesehen.

Im Rahmen der Anmeldung ist weiterhin eine Reaktionsluft-Aufbereitungsanlage offenbart.

Diese umfasst einen Druckluftbehälter, der von einem Kompressor mit Reaktionsluft gespeist wird.

Ausgangsseitig des Druckluftbehälters ist eine Lufttrocknungseinrichtung befindlich, welche mit einer Filteranordnung in Verbindung steht.

Filterausgangsseitig ist eine regelbare Temperier-, insbesondere Heizeinrichtung angeordnet, die über eine Durchflussmess- und Stelleinrichtung mit mindestens einem Luftverteiler in Verbindung steht.

Der Luftverteiler kann die bereits erwähnten Lochrohre oder Lochschläuche umfassen.

Ausgestaltend weisen sowohl die Lufttrocknungseinrichtung als auch der Druckluftbehälter einen niveaugeregelten Kondensatableiter auf.

Die Heizeinrichtung bzw. die Einrichtung zum Temperieren kann als Rohrheizer ausgebildet sein.

Die Filteranordnung umfasst zweckmäßigerweise einen Mikro- und Sterilfilter, um einen schädigenden Einfluss auf die für die Bildung der Cellulose notwendigen Bakterien zu vermeiden.

Weiterhin ausgestaltend kann die Lufttrocknungseinrichtung als Kälte-Drucklufttrockner ausgebildet werden.

Die Erfindung soll nachstehend anhand eines Ausführungsbeispiels sowie unter Zuhilfenahme von Figuren näher erläutert werden.

Hierbei zeigen:
- Fig. 1: eine schematische Darstellung der erfindungsgemäßen Vorrichtung zur Herstellung bakteriell synthetisierter Cellulose oder cellulosehaltigem Flächenmaterial in teilweise weggebrochener Seitenansicht und
- Fig. 2: ein Blockschaltbild der erfindungsgemäßen Reaktionsluft-Aufbereitungsanlage.

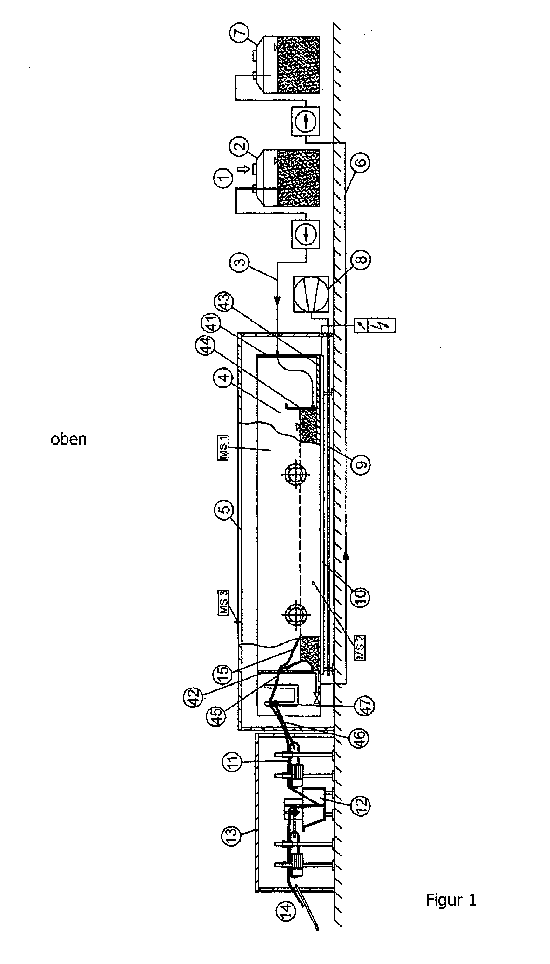

Gemäß der Darstellung nach Fig. 1 umfasst die Vorrichtung zur Herstellung bakteriell synthetisierter Cellulose oder cellulosehaltigem Flächenmaterial einen Behälter für Nährmittellösung. Dieser Behälter 2 wird mit Nährmittel einerseits vorkultivierten Baktierenstämmen (symbolisiert mit dem Bezugszeichen 1) versorgt.

Über eine entsprechende Durchflussmess- und Regeleinrichtung wird die nährstoffreiche Lösung in den eigentlichen Reaktionsraum eingeführt. Diese Zuführung kann über eine Schlauchverbindung 3 realisiert werden.

Im Inneren einer Einhausung 5 befindet sich als Reaktionsgefäß eine Reaktionswanne, die den Reaktionsraum 4 bildet.

Wie aus der Fig. 1 ersichtlich, ist das Reaktionsgefäß als langgestreckter Wannenkörper mit einem ersten Ende 41 und einem zweiten Ende 42 ausgebildet.

Eine Öffnung am ersten Ende 41 nimmt die Zuführung, z.B. einen Schlauch der nährstoffreichen Lösung auf. Am Boden 43 der Reaktionswanne befindet sich in der Nähe des ersten Endes 41 ein Verteilkörper 44.

Dieser Verteilkörper 44 weist Bohrungen und Anschlussstutzen für die Zuführung 3 auf. Der Verteilkörper sichert eine homogene gleichmäßige und strömungsarme Verteilung der nährstoffreichen Lösung. Die Bohrungen im Verteilkörper 44 liegen bevorzugt unterhalb des Nährstoffflüssigkeitsspiegels.

Eine Rückführung 6 der nährstoffarmen Lösung aus dem Reaktionsraum erfolgt durch entsprechende Öffnungen in der Reaktionswanne, die sich im Bereich des zweiten Endes 42 befinden. Ein Absperrventil ermöglicht die gezielte, gesteuerte Rückführung der Lösung hin zu einem entsprechenden Aufnahmebehälter 7.

Eine Anlage zur Erzeugung und Aufbereitung von Reaktionsluft 8 stellt diese bereit, wobei über Rohrschläuche 9, die sich bevorzugt beidseitig der Reaktionswanne befinden, für eine turbulenzarme Zuführung und Verteilung der Reaktionsluft gesorgt wird.

Im Bereich des Wannenbodens 43 ist noch ein System zum Temperieren, insbesondere zum Beheizen 10 des Reaktionsraums 4 vorhanden.

Die Reaktionswanne ist von einer Einhausung 5 umgeben. Die Einhausung 5 weist an ihrem zum Abzugs- und Fördermodul gerichteten Ende entsprechende Öffnungen auf, so dass das gewonnene Nanocellulose-Vlies 15 entnommen werden kann.

Am zweiten Ende 42 des Wannenkörpers ist eine Gleitschräge 45 ausgebildet, die das Abheben und Heraustransportieren des Nanocellulose-Vlieses 15 erleichtert.

Im mit einer separaten Kapselung 13 versehenen Abzugs- und Fördermodul befindet sich eine Spül- und Reinigungswanne 12. Der Modul umfasst darüber hinaus ein Abzugssystem 11 für das Reaktionsprodukt. Dieses Abzugssystem kann ein Band, insbesondere ein Noppenband aufweisen, das motorisch angetrieben wird. Mit 14 ist der Fertigproduktaustrag zur Weiterbehandlung, d.h. zur Trocknung und Konfektionierung symbolisiert.

An der Messstelle MS1 erfolgt eine Bestimmung der Temperatur der Reaktionsluft. An den Messstellen MS2 und MS3 wird die Sauerstoffkonzentration in der Reaktionslösung bzw. in der Reaktionsluft bestimmt.

Das Heizsystem wird über eine symbolisierte Stromquelle (Elektropfeil) mit Energie versorgt.

Der Abzugs- und Fördermodul kann einen verstellbaren Abzugsarm 46 umfassen, welcher an seinem zur Wanne reichenden Ende eine Abzugswalze oder mindestens ein Abzugsritzel 47 besitzt.

Das Abzugsritzel 47 nimmt einen unterhalb des Flächenmaterials befindlichen flexiblen, flächenförmigen Produktstützkörper (nicht gezeigt) auf. Hierdurch gelingt es, das eigentliche Produkt kräfte- und verformungsfrei aus dem Reaktionsraum zu entfernen. Dieser Stützkörper kann z.B. als ein eigensteifes Maschennetz ausgeführt werden.

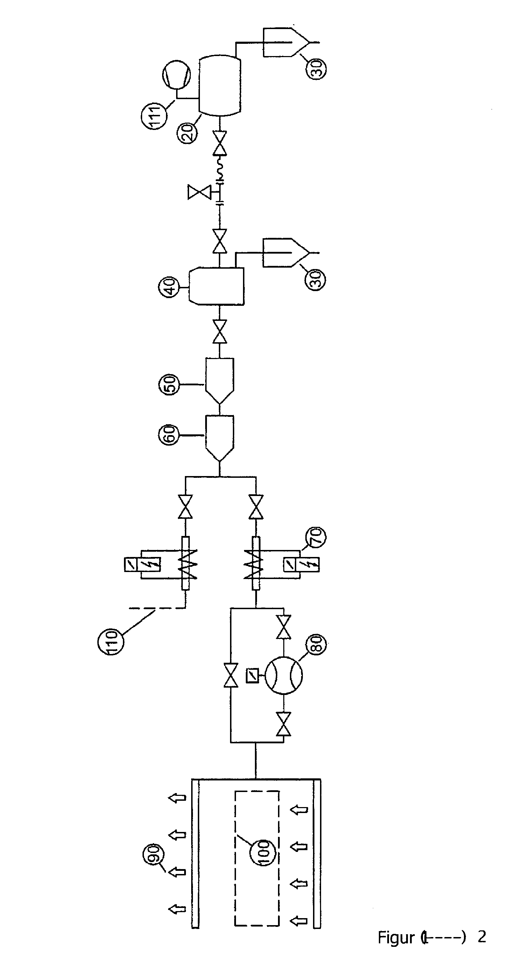

Die Reaktionsluft-Aufbereitungsanlage 8 stellt sich unter Hinweis auf das Blockschaltbild gemäß Fig. 2 wie folgt dar.

Ein ölfrei verdichtender Kolbenkompressor 111 saugt Umgebungsluft an und befördert diese in einen Druckluftbehälter 20.

Der Druckluftbehälter 20 weist einen bevorzugt elektronisch niveaugeregelten Kondensatableiter 30 auf.

Ausgangsseitig des Druckluftbehälters 20 ist über eine Ventilanordnung eine Verbindung zu einem Kälte-Drucklufttrockner 40 hergestellt. Auch dieser Kälte-Drucklufttrockner 40 besitzt eine niveaugeregelte Kondensatableiteinrichtung 30.

Wiederum über ein Ventil gelangt die getrocknete Reaktionsluft auf eine Filteranordnung. Diese umfasst z.B. einen Mikrofilter 50 sowie einen Sterilfilter 60.

Eine Verteilanordnung führt dann die Reaktionsluft zu den Reaktionswannen 100 und in dort vorgesehene Mittel 90 zur turbulenzarmen Verteilung der Reaktionsluft.

In der Fig. 2 ist hier nur eine solche Reaktionswanne 100 angedeutet. Eine zweite Reaktionswanne 110 kann sich jedoch anschließen.

Eine Rohrbeheizung 70 ist elektrisch steuerbar, um eine möglichst konstante Temperatur der Reaktionsluft zu gewährleisten.

Über eine Durchflussmesseinrichtung 80 mit Stellventilen wird die Reaktionsluft, wie erläutert, zum System 90 zur turbulenzarmen Verteilung geleitet. Das System 90 kann Schläuche mit einer Vielzahl von radialen Bohrungen aufweisen, so dass die Reaktionsluft sich gleichmäßig entlang und in der Umgebung der Reaktionswanne 100 verteilt.

Die Kultivierung der Cellulose unter Zuhilfenahme der vorgestellten technischen Komponenten sei nachstehend beschrieben.

Jeweils der an der Grenzfläche des Kulturmediums zur Reaktionsluft in einer definierten Dicke fertig kultivierte Teil der bakteriell synthetisierten Cellulose wird als flächenförmiges Vlies- oder folienartiges Material ohne Abtrennung von dem noch nicht fertig kultivierten Teil des erhaltenen Materials als Band aus der Reaktionswanne, die als Kulturgefäß dient, entnommen.

Der noch nicht fertig kultivierte Teil des cellulosehaltigen Materials verbleibt noch im Reaktionsgefäß und kann dort quasi ungestört von der Entnahme weiterkultiviert werden. Nach Erreichen einer definierten Dicke wird dieser kultivierte Teil, wiederum unabgetrennt von dem bereits entnommenen Teil des cellulosehaltigen Materials sowie ebenfalls unabgetrennt von dem noch im Reaktionsgefäß verbleibenden Teil in Bandform aus dem Reaktionsgefäß entnommen.

Die jeweils fertig kultivierte Cellulose wird dann nach der Entnahme aus dem Reaktionsgefäß einem Medium ausgesetzt, das einen weiteren Kultivierungsprozess verhindert.

Aus diesen Erläuterungen ist ersichtlich, dass das aus der jeweils fertig kultivierten bakteriell synthetisierten Cellulose bestehende Band kontinuierlich aus dem Reaktionsgefäß entnommen werden kann. Diese Entnahme kann kontinuierlich, quasi kontinuierlich, aber auch schrittweise erfolgen.

Durch die Steuerung der Bandgeschwindigkeit bezüglich der Entnahme aus dem Reaktionsgefäß ist die Dicke der jeweils fertig kultivierten Cellulose vorgebbar. Zur Unterstützung der Entnahme des Flächenmaterials können entsprechende Mitnehmer vorgesehen sein, die bevorzugt formschlüssig die Förderung des Bandes unterstützen.

Der flexbile, flächige Stützkörper kann in das cellulosehaltige Material einkultiviert werden. Bei der Entnahme und dem Transport des Bandes werden nicht zu vermeidende Kräfte dann vom flächigen Stützkörper und nicht vom Vlies aufgenommen, so dass dessen Struktur erhalten bleibt.

### Bezugszeichenliste

- 1: Beistellung der Nährmittellösung und der vorkultivierten Bakterienstämme
- 2: Behälter für Nährmittellösung
- 3: Zuführung der nährstoffreichen Lösung
- 4: Reaktionsraum (Reaktionswanne)
- 5: Einhausung
- 6: Rückführung der nährstoffarmen Lösung
- 7: Behälter für nährstoffarme Lösung
- 8: Reaktionsluft-Erzeugungsanlage
- 9: Mittel zur turbulenzarmen Zuführung und Verteilung der Reaktionsluft
- 10: Heizsystem zur Temperierung des Reaktionsraums
- 11: Abzugssystem für das Reaktionsprodukt
- 12: Spül- und Reinigungswanne
- 13: Einhausung der Spül- und Reinigungswanne
- 14: Fertigproduktaustrag
- 15: Nanocellulose-Vlies
- MS1: Messstelle der Temperatur der Reaktionsluft
- MS2: Messstelle der Sauerstoffkonzentration in der Reaktionslösung
- MS3: Messstelle der Sauerstoffkonzentration in der Reaktionsluft
- 41: erstes Ende
- 42: zweites Ende
- 43: Boden
- 44: Verteilkörper
- 45: Gleitschräge
- 46: Abzugsarm
- 47: Abzugsritzel
- 111: Kolbenkompressor
- 20: Druckluftbehälter
- 30: elektronisch niveaugeregelter Kondensatableiter
- 40: Kälte-Drucklufttrockner
- 50: Mikrofilter
- 60: Sterilfilter
- 70: Rohrbeheizung
- 80: Durchflussmesser mit Stellventil
- 90: System zur turbulenzarmen Verteilung der Reaktionsluft
- 100: Reaktionswanne
- 110: weitere Reaktionswanne

## Patentansprüche

1. Vorrichtung zur Herstellung bakteriell synthetisierter Cellulose oder cellulosehaltigem Flächenmaterial, umfassend ein wannenartiges Reaktionsgefäß zur Aufnahme einer Nährlösung, Mittel zur Nährlösungszuführung und Temperierung des Reaktionsgefäßes sowie zur Sauerstoffversorgung,
das Reaktionsgefäß einen langgestreckten Wannenkörper (4) mit einem ersten (41) und einem zweiten (42) Ende aufweist, wobei am ersten Ende (41) ein Zulauf (3) für eine inhaltsreiche Nährstofflösung und am zweiten, gegenüberliegenden Ende (42) ein Ablauf (6) für inhaltsarme Nährstofflösung vorgesehen ist,
weiterhin am zweiten Ende (42) des Wannenkörpers (4) eine Gleitschräge (47) für das abzuziehende Flächenmaterial (15) ausgebildet ist und sich außerhalb des zweiten Endes (42) des Wannenkörpers (4) ein Abzugs- und Fördermodul befindet, welcher mindestens ein motorisch angetriebenes Band (11) sowie eine Spül- und Reinigungswanne (12) für das abgezogene Flächenmaterial (15) aufweist,
**dadurch gekennzeichnet, dass**
mindestens der Zulauf (3) für die inhaltsreiche Nährstofflösung unterhalb des Nährstofffüllspiegels im Wannenkörper (4) befindlich ist, der Abzugs- und Fördermodul einen verstellbaren Abzugsarm (46) aufweist, welcher an seinem zur Wanne (4) reichenden Ende eine Abzugswalze oder mindestens ein Abzugsritzel (47) besitzt, die Walze oder das Ritzel (47) mit einer Antriebseinrichtung in Verbindung steht und die Walze oder das Ritzel (47) einen unterhalb des Flächenmaterials (15) befindlichen, flexiblen, flächigen Produktstützkörper aufnimmt und/oder diesen zum Zweck des Transports kontaktiert.

2. Vorrichtung nach Anspruch 1,
**dadurch gekennzeichnet, dass**
der Zulauf einen Verteilkörper (44) zum Erzeugen eines definierten Nährstofflösungs-Volumenstroms aufweist.

3. Vorrichtung nach Anspruch 2,
**dadurch gekennzeichnet, dass**
der Verteilkörper (44) am Wannenboden (43) befindlich ist und einen in Wannenquerrichtung verlaufenden Steg mit Bohrungen und Schlauchanschlussstutzen aufweist.

4. Vorrichtung nach einem der vorangegangenen Ansprüche,
**dadurch gekennzeichnet, dass**
die Gleitschräge (45) eine Schrägfläche umfasst, deren Verlauf sich von unterhalb des Nährstofffüllspiegels bis zu einer Öffnung in der Wanne (4) oberhalb des Nährstofffüllspiegels erstreckt.

5. Vorrichtung nach Anspruch 1,
**dadurch gekennzeichnet, dass**
der Stützkörper als ein eigensteifes Netzmaterial ausgebildet ist.

6. Vorrichtung nach einem der vorangegangenen Ansprüche,
**dadurch gekennzeichnet, dass**
mindestens der Wannenkörper (4) von einer Umhausung (5) umgeben ist, welche mindestens in ihren Breitenabmessungen größer als der Wannenkörper (4) ist, wobei im jeweiligen seitlichen Zwischenraum Lochrohre oder Lochschläuche (9) zur turbulenzarmen Zuführung von Reaktionsluft vorhanden sind.

7. Vorrichtung nach einem der vorangegangenen Ansprüche,
**dadurch gekennzeichnet, dass**
im, am und/oder unterhalb des Wannenkörpers (4) Mittel zum Beheizen (10) desselben vorgesehen sind.

8. Vorrichtung nach Anspruch 7,
**dadurch gekennzeichnet, dass**
die Mittel zum Beheizen eine Heizmatte (10) umfassen.

9. Vorrichtung nach einem der vorangegangenen Ansprüche,
**dadurch gekennzeichnet, dass**
das motorisch angetriebene Band ein Endlos-Noppenband ist.

10. Vorrichtung nach einem der vorangegangenen Ansprüche,
**dadurch gekennzeichnet, dass**
Mittel zum Messen der Sauerstoffkonzentration in der Reaktionsluft (MS3) sowie Mittel zum Messen der Sauerstoffkonzentration in der Reaktionslösung (MS2) vorgesehen sind.

## Claims

1. A device for producing bacterially synthesized cellulose or cellulose containing planar material, comprising:
a tub shaped reaction vessel for receiving a nutritious solution;
devices for supplying the nutritious solution and tempering the reaction vessel and for supplying oxygen,
wherein the reaction vessel includes an elongated tub element (4) including a first end (41) and a second end (42),
wherein an inlet (3) for a high content nutritious material solution is provided at the first end (41) and an outlet (6) for low content nutritious solution is provided at the second opposite end (42),
wherein a slanted slide (47) for the flat material (15) to be pulled off is provided at the second end (42) of the tub element (4) and an extraction- and feed module is provided outside of the second end (42) of the tub element (4),
wherein the extraction- and feed module includes at least one motor driven belt (11) and a flushing- and cleaning tub (12) for the pulled off flat material (15),
wherein at least the inlet (3) for the high content nutritious solution is arranged below the nutritious material filling level in the tub element (4),
wherein the extraction and feed module includes an adjustable extraction arm (46) which includes a pull off roller or at least one pull off sprocket (47) at its end oriented towards the tub (4),
wherein the roller or the sprocket (47) is connected with a drive device and the roller or the sprocket (47) receives a flexible flat product support element arranged below the flat material (15), and/or
wherein the roller or the sprocket (47) contacts the flexible flat product support element for transportation.

2. The device according to claim 1, wherein the inlet includes a distributor element (44) for generating a defined nutritious solution volume flow.

3. The device according to claim 2,
wherein the distributor element (44) is arranged at the tub base (43) and includes a bar with bore holes and hose connection spouts,
wherein the bar extends in a transversal direction of the tub.

4. The device according to one of the preceding claims,
wherein the slanted slide (45) includes a slanted surface which extends from below a nutritious solution filling level to an opening in the tub (4) above the nutritious solution filling level.

5. The device according to claim 1, wherein the support element is configured as a mesh material that is stiff in itself.

6. The device according to one of the preceding claims,
wherein at least the tub element (4) is enclosed by a housing element (5) whose dimensions are greater at least in width than the width of the tub element (4),
wherein perforated tubes or perforated hoses (9) are provided in a respective lateral intermediary space for supplying low turbulence reaction air.

7. The device according to one of the preceding claims, wherein devices (10) for heating the tub element (4) are provided in at and/or below the tub element (4).

8. The device according to claim 7, wherein the heating devices include a heating mat (10).

9. The device according to claim one of the preceding claims, wherein the motor driven band is an endless knob band.

10. The device according to one of the preceding claims, wherein devices for measuring an oxygen concentration in the reaction air (MS3) are provided and devices for measuring an oxygen concentration in the reaction solution (MS2) are provided.

## Revendications

1. Appareil pour la production par synthèse bactérienne de cellulose ou de matériau surfacique contenant de la cellulose, comprenant un récipient de réaction semblable à une cuve pour recevoir une solution nutritive, des moyens pour l'admission de solution nutritive et pour tempérer le récipient de réaction ainsi que pour l'alimentation d'oxygène, le récipient de réaction comprenant un corps en cuve allongé (4) avec une première extrémité (41) et seconde extrémité (42), dans lequel il est prévu à la première extrémité (41) une admission (3) pour une solution nutritive riche, et à la seconde extrémité opposée (42) une évacuation (6) pour une solution nutritive pauvre,
à la seconde extrémité (42) du corps en cuve (4) est ménagée une pente de coulissement (47) pour le matériau surfacique à peler (15), et un module de pelage et de convoyage se trouve à l'extérieur de la seconde extrémité (42) du corps en cuve (4), ce module comprenant au moins une bande à entraînement motorisé (11) ainsi qu'une cuve de rinçage et de nettoyage (12) pour le matériau surfacique pelé (15),
**caractérisé en ce que**
au moins l'admission (3) pour la solution nutritive riche se trouve au-dessous du niveau de remplissage du produit nutritif dans le corps en cuve (4), le module de pelage et de convoyage comprend un bras de pelage réglable (46) qui possède, à son extrémité tournée vers la cuve (4), un cylindre de pelage ou au moins un pignon de pelage (47), le cylindre ou le pignon (47) est en liaison avec un système d'entraînement, et le cylindre ou le pignon (47) reçoit un corps de soutien de produit surfacique flexible, qui se trouve au-dessous du matériau surfacique (15) et/ou vient en contact avec celui-ci dans le but du transport.

2. Appareil selon la revendication 1,
**caractérisé en ce que** l'admission comprend un corps de distribution (44) pour générer un courant volumétrique défini de solution nutritive.

3. Appareil selon la revendication 2,
**caractérisé en ce que** le corps de distribution (44) se trouve au fond (43) de la cuve et comporte un une barrette, s'étendant en direction transversale à la cuve, avec des perçages et des pipes de raccordement pour tuyaux.

4. Appareil selon l'une des revendications précédentes,
**caractérisé en ce que** la pente de coulissement (45) comporte une surface oblique dont le tracé s'étend depuis au-dessous du niveau de remplissage de produit nutritif jusqu'à l'ouverture dans la cuve (4) au-dessus du niveau de remplissage de produit nutritif.

5. Appareil selon la revendication 1,
**caractérisé en ce que** le corps de soutien est réalisé comme un matériau en forme de filet présentant une rigidité propre.

6. Appareil selon l'une des revendications précédentes,
**caractérisé en ce qu'**au moins le corps de cuve (4) est entouré par un habillage (5), qui, au moins dans ses dimensions en largeur, est plus grand que le corps de cuve (4), et dans l'espace latéral intermédiaire respectif il est prévu des tubes à trous ou des tuyaux à trous (9) pour l'admission d'air de réaction d'une manière à présenter peu de turbulences.

7. Appareil selon l'une des revendications précédentes,
**caractérisé en ce qu'**il est prévu dans, sur et/ou au-dessous du corps de cuve (4) des moyens (10) pour chauffer celui-ci.

8. Appareil selon la revendication 7,
**caractérisé en ce que** les moyens de chauffage comprennent une nappe chauffante (10).

9. Appareil selon l'une des revendications précédentes,
**caractérisé en ce que** la bande à entraînement motorisé est une bande à doigts sans fin.

10. Appareil selon l'une des revendications précédentes,
**caractérisé en ce qu'**il est prévu des moyens pour mesurer la concentration en oxygène dans l'air de réaction (MS3), ainsi que des moyens pour mesurer la concentration en oxygène dans la solution de réaction (MS2).
